# EUROPEAN PATENT SPECIFICATION

(11) **EP 0 784 963 B1**
(45) Date of publication and mention of the grant of the patent: **06.02.2002**
(21) Application number: 96117815.9
(22) Date of filing: 07.11.1996
(51) Int. Cl.: A61B 17/70

(54) **Connecting element for a stabilizing system for a human spine**
Verbindungselement für ein Stabilisierungssystem für eine menschliche Wirbelsäule
Elément de raccordement pour un système de stabilisation pour la colonne vertébrale

(30) Priority: 18.01.1996 DE 29600784 U
(43) Date of publication of application: 23.07.1997
(73) Proprietor: Howmedica GmbH, D-24232 Schönkirchen (DE)
(72) Inventor: Metz-Stavenhagen, Peter, Dr., 34537 Bad Wildungen (DE); Robioneck, Bernd, 24111 Preetz (DE)
(74) Representative: Patentanwälte Hauck, Graalfs, Wehnert, Döring, Siemons

(56) References cited:
- WO-A-96/28104
- DE-U- 9 409 123
- US-A- 4 773 402
- US-A- 4 805 602
- US-A- 5 261 909

## Description

The invention relates to a connecting element for fixing a rod at a pedicle screw or the like for a stabilizing system for a human spine, the element having a first section for receiving the pedicle screw and a second section for receiving and fixing by clamping the rod passed therethrough.

A connecting element comprising the features specified in the preamble of claim 1 is disclosed in US-A-5 261 909.

Stabilizing systems for the human spine usually comprise rods which are dorsally secured at the spine with pedicle, sacral or iliac screws for distraction or compression of the spine or are secured with hooks grasping around the transverse processes of the vertebrae. For connecting the screws and the hooks with the rod, connecting elements are utilized. For instance, connecting elements are known (ISOLA^{(R)} system), wherein a first section for receiving a pedicle screw or the like is formed as an elongated hole being led onto the head of the screw and secured with a nut. A second section for receiving the rod comprises a conically formed opening in which the rod is put and fixed by a screw which presses against the rod from the top with the end of the shank.

A disadvantage of these known connecting elements is that two securing means have to be operated in order to connect the rod with the screws inserted into the spine, namely the nut for securing the connecting elements at a screw in the spine and the screw for fixing the rod in the receiving opening of the connecting elements.

Thus, the object of the invention is to provide a connecting element for connecting a screw in the spine and a stabilizing rod, the element being easily and time-savingly securable.

This object is achieved by the features of the claim 1.

In the connecting element according to the invention, the second section serving for receiving the rod is part of a tension member which further has a thread shank. A sleeve having a through-opening for the shank is arranged around the second section in order that the sleeve can be slidingly moved parallel to the shank axis. In the wall of the sleeve two openings opposed to each other or recesses closed towards the shank are formed for the rod. Furthermore, the connecting element comprises a nut which can be screwed onto the shank in order to fix the head of the pedicle screw or of a similar screw, which is to be screwed into the spine, at the sleeve. Simultaneously with the fixation of the screw the sleeve is so far displaced towards the end of the tension member being opposed to the shank that the rod being inserted into the receptacle is secured between the walls of the receptacle and the openings or the recesses of the sleeve by clamping.

This connecting element has the advantage both the rod can be fixed in the receptacle of the tension member and the connecting element can be secured at the screw head by a single securing process, namely the tightening of the nut.

The second section serving for receiving the rod can comprise a circular receiving opening for the rod. Preferably, the center thereof lies on the longitudinal axis of the connecting element. Thereby, a turning of the connecting element or at least a part of the rod which usually is flexible around the longitudinal axis of the connecting element during fixation is more easily possible.

Alternatively, it can be provided that the second section comprises a recess being open transversely to the shank axis. This can be a longitudinal recess with a semi-circular end. This form of the receptacle has the advantage that the rod can be put in and does not have to be pushed through the opening which can save time when using a longer rod which is to be secured at several connecting elements.

The recesses of the sleeve can be open towards the free end thereof, whereby a sliding onto the second section from the side facing the shank is possible if a rod is located in the second section. Preferably, the receptacle for the rod and the recesses of the sleeve are formed so that an opening is formed which extends transversely through sleeve and tension member, with the rod being fixed by clamping in the opening.

The sleeve can comprise a toothing around the throughgoing opening of the shank for engaging with a toothing of the pedicle screw head. Especially if such a toothing is provided, it is advantageous if the sleeve is round in cross section and turnable on the tension member around the shank axis thereof.

The sleeve can be slidable onto the shank of the tension member, whereby the sleeve through-opening for the shank is smaller than the transverse dimensions of the second section so that the sleeve upon a sliding in the direction of the second section finally strikes it if no rod is inserted into the receptacle. Therefore, the second section preferably can comprise an inwardly inclined region within the transition region towards the shank, the inclined region constituting a stop face for the corresponding inner surface of the sleeve.

For engaging with an opening or recess in the pedicle screw head, the nut can have a collar.

In the following the invention will be illustrated with reference to drawings.
- Fig. 1: shows a connecting element in elevational view, partly in sectional view, and schematically a pedicle screw.
- Fig. 2: shows the connecting element of Fig. 1 seen in direction of the arrow A, whereby the pedicle screw of Fig. 1 is not shown and additionally a received rod is shown.
- Fig. 3: shows a further embodiment of the connecting element according to the invention in elevational view and partly in sectional view.
- Fig. 4: shows the connecting element of Fig. 3, whereby the sleeve is located in a position which allows for putting the rod into the connecting element.

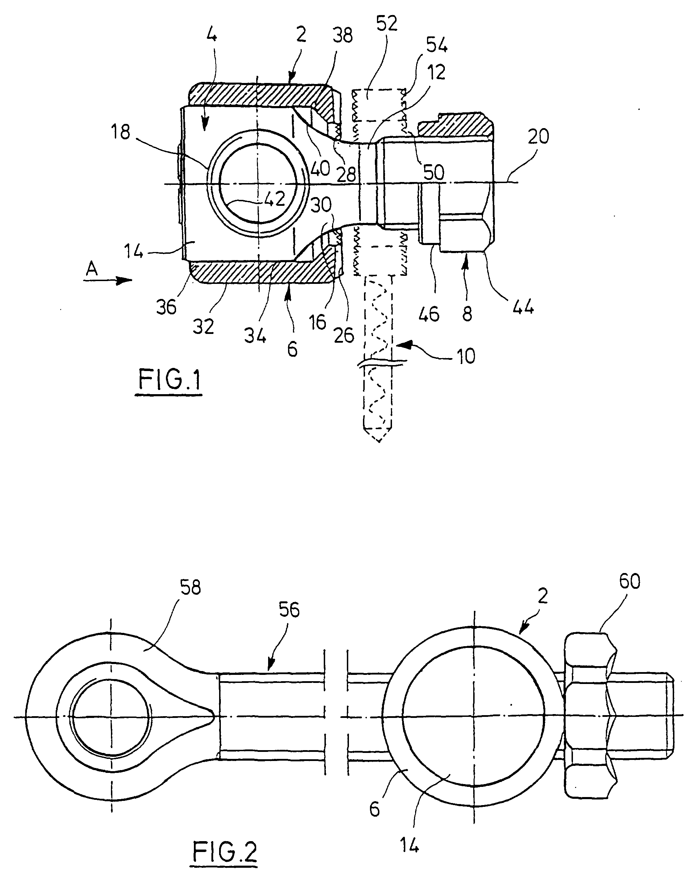

Fig. 1 shows a connecting element 2 which consists of a tension member 4, a sleeve 6 and a nut 8. Furthermore, Fig. 1 shows schematically a pedicle screw 10 at which the connecting element 2 is secured.

The tension member 4 comprises a first section 12 for receiving the pedicle screw 10 and a second section 14 for receiving and fixing a rod (not shown) by clamping for stabilizing a human spine. The first section 12 is a thread shank onto which the nut 8 can be screwed. The second section 14 attaches to the shank 12 via a curved transition region 16 and has larger transverse dimensions than the shank 12. As is to be seen in Fig. 2, the second section has a cylindrical shape. The second section 14 includes a circular through-receiving opening 18 which extends transversely relative to the longitudinal axis 20 of the connecting member 2.

The sleeve 6 is also cylindrical. The sleeve 6 includes a through-opening 28 for the shank 12 in an end plane 26 facing the shank. A toothing 30 is formed around the through-opening 28. The cylindrical inner wall surface 34 of the sleeve 6 nearly matchingly, however, glidingly receives the cylindrical outer wall surface 36 of the second section 14. The sleeve wall 32 includes a conical ring surface 38 which is inclined towards the opening 30 and which is parallel to the surface area 40 of the transition region 16 of the first section 14. In the sleeve wall 32 two circular through-openings 42 being opposed to each other are formed which in the position of the sleeve 6 shown in Fig. 1 are concentric with the opening 18 of the tension member 4, however, which have a slightly smaller diameter.

The nut 8 includes an essentially hexagonal head 44 which is adjoined by a collar 46 towards the shank, whereby the nut 8 engages with the collar with a circular opening 50 in the head 52 of the pedicle screw 10.

On its outer surface the head 52 of the pedicle screw 10 includes a toothing 54 around the opening 50 in order to engage with the toothing 30 of the sleeve 6.

The connecting element 2 is applied as follows. At first it is passed with the shank 12 through the opening 50 of the pedicle screw head 52. Thereafter, the nut 8 is screwed onto the shank 12 so far until the opening 18 of the tension member 4 and the openings 42 of the sleeve 6 are concentric. Then, the rod to be secured (not shown) is passed through the openings and the nut 8 is tightened so that the sleeve 6 is fixed against the head 52 of the pedicle screw 10 and simultaneously the sleeve is displaced towards the shank 12 relative to the tension member 4 so far until the rod between the opening 18 and 42 is clamped.

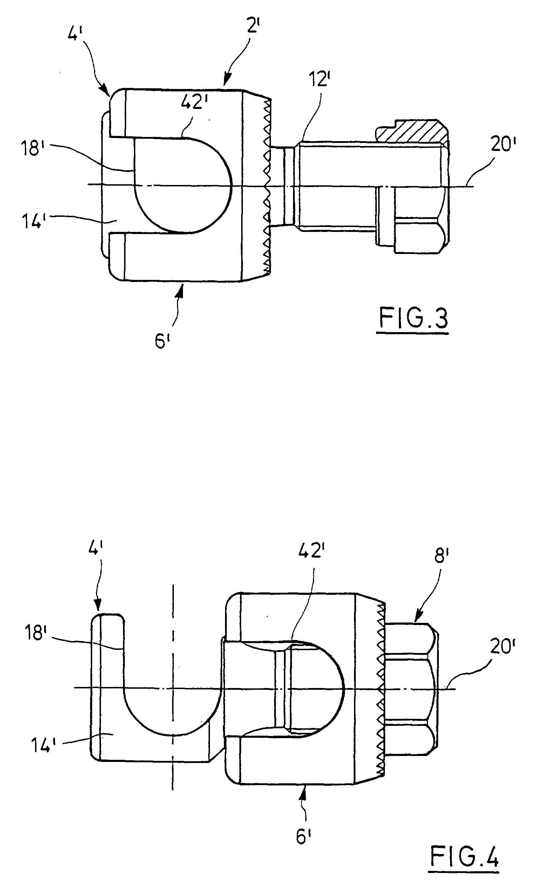

Fig. 2 shows the connecting element 2 of Fig. 1 in direction of the arrow A. Therein, the pedicle screw 10 is not shown. Additionally, a thread rod 56 fixed in the connecting element 2 for stabilizing a spine is shown. At an end, the rod 56 has an eye-shaped opening 58 for securing at an iliac screw. The rod 56 is passed transversely relative to the longitudinal direction 20 of the connecting elements 2 through the openings 42 of the sleeve 6 and the opening 18 of the second section. A nut 60 is screwed on at the end of the rod 56 being opposed to the opening 58. The nut 60 is screwed against the connecting element 2. As a result, a traction can be applied to the rod 56 and thus to the screw passed through the opening 58 before the rod 56 is fixed in the connecting element 2. The Figs. 3 and 4 show a further embodiment 2' of the connecting element according to the invention. The connecting element 2' differs from the connecting element 2 shown in Fig. 1 essentially in that instead of the opening 18 of the connecting element 2, the connecting element 2' is provided with a recess 18' and further instead of the openings 42 with recesses 42'.

The recess 18' is located in a second section 14' of a tension member 4' and is opened transversely relative to an axis 20' of a thread shank 12' (Fig. 4). Apart from the open side, the recess 18' is formed like an elongated hole which is oriented perpendicular relative to the longitudinal axis 20'.

The recesses 42' are provided in a sleeve 6' and have a shape which is essentially the same as that of the recess 18', however, they are open towards the end of the sleeve 6' opposed to the shank 12', whereby the longitudinal axes of the recesses 42' are parallel relative to the shank axis 20'.

In Fig. 3 the sleeve 6' is slided over the second section 14' such that a closed opening for a rod is provided by superposition of the recesses 18' and 42'. In Fig. 4 the sleeve 6' is shown displaced towards the shank 12' so that the recess 18' of the tension member 4' is not covered and a rod can be put into the recess 18' through its open end. Subsequently, the rod can be fixed by clamping as the sleeve 6' is slided towards the second section 14' and the nut 8' is tightened as explained above.

## Claims

1. Connecting element (2) for fixing a rod (56) at a pedicle screw (10) for a stabilizing system for a human spine, comprising
- a tension member (4, 4') with
- a first section comprising a threaded shank (12, 12') for receiving a head of a pedicle screw and
- a second section (14, 14') comprising an opening (18, 18') for receiving and clamping said rod,
- a sleeve (6) slidingly arranged on said second section (14),
- said sleeve having a through-opening (28) for said threaded shank and having two openings (42) opposed to each other or two recesses (42') being closed toward the shank for receiving said rod, and
- a nut (8, 8') being screwable onto the shank for fixing the sleeve (6, 6') at the head (52) of the pedicle screw (10),
**characterized in that** the through-opening (28) of the sleeve (6, 6') for the shank is smaller than the transverse dimensions of the second section of the tension member.

2. Connecting element according to claim 1, **characterized in that** the second section (14) comprises a circular receiving opening.

3. Connecting element according to claim 1, **characterized in that** the second section (14') comprises a recess (18') opened transversely relative to the shank axis (20').

4. Connecting element according to one of the preceding claims, **characterized in that** the recesses (42') of the sleeve (6') are open towards the free end of the sleeve.

5. Connecting element according to one of the preceding claims, **characterized in that** the sleeve (6, 6') comprises a toothing (30) around the through-opening (28) for the shank (12, 12') for engagement with a toothing (54) of the pedicle screw head (52).

6. Connecting element according to one of the preceding claims, **characterized in that** the sleeve (6, 6') is round in cross section.

7. Connecting element according to one of the preceding claims, **characterized in that** in the region (16) of transition to the shank (12, 12') the second section (14, 14') comprises a preferably inwardly inclined region which constitutes a stop face for an inner surface (38) of the sleeve (6, 6').

8. Connecting element according to one of the preceding claims, **characterized in that** the nut (8, 8') comprises a collar (46) for engagement with an opening (50) of the pedicle screw head (52).

## Patentansprüche

1. Verbindungselement (2) zur Festlegung einer Stange (56) an einer Pedikelschraube (10) für ein Stabilisierungssystem für eine menschliche Wirbelsäule, mit
- einem Zuganker (4, 4') mit
- einem ersten Abschnitt, der einen Gewindeschaft (12, 12') zur Aufnahme eines Kopfs einer Pedikelschraube und
- einem zweiten Abschnitt (14, 14') mit einer Öffnung (18, 18') zur Aufnahme und zur Klemmung der Stange besitzt,
- eine Hülse (6), die gleitend auf dem zweiten Abschnitt (14) angeordnet ist,
- wobei die Hülse eine Durchgangsöffnung (28) für den Gewindeschaft und zwei einander gegenüberliegende Öffnungen (42) oder zwei zum Schaft hin geschlossene Aussparungen (42') für die Stange besitzt, und
- eine auf den Schaft schraubbare Mutter zur Festlegung der Hülse (6, 6') an dem Kopf (52) der Pedikelschraube (10),
- **dadurch gekennzeichnet, dass** die Durchgangsöffnung (28) der Hülse (6, 6') für den Schaft kleiner als die Querabmessungen des zweiten Abschnitts des Zugankers sind.

2. Verbindungselement nach Anspruch 1, **dadurch gekennzeichnet, dass** der zweite Abschnitt eine kreisförmige Aufnahmeöffnung aufweist.

3. Verbindungselement nach Anspruch 1, **dadurch gekennzeichnet, dass** der zweite Abschnitt (14') eine quer zur Schaftachse (20') geöffnete Aussparung (18') besitzt.

4. Verbindungselement nach einem der vorangehenden Ansprüche, **dadurch gekennzeichnet, dass** die Aussparungen (42') der Hülse (6') zu deren freiem Ende hin offen sind.

5. Verbindungselement nach einem der vorhergehenden Ansprüche, **dadurch gekennzeichnet, dass** die Hülse (6, 6') um die Durchgangsöffnung (28) für den Schaft (12, 12') eine Verzahnung (30) aufweist, zum Eingriff mit einer Verzahnung (54) des Pedikelschraubenkopfes (52).

6. Verbindungselement nach einem der vorhergehenden Ansprüche, **dadurch gekennzeichnet, dass** die Hülse (6, 6') einen runden Querschnitt besitzt.

7. Verbindungselement nach einem der vorhergehenden Ansprüche, **dadurch gekennzeichnet, dass** in dem Übergangsbereich (16) zum Schaft (12, 12') der zweite Abschnitt (14, 14') eine bevorzugt nach innen geneigten Bereich aufweist, der eine Anschlagfläche für eine Innenfläche (38) der Hülse (6, 6') bildet.

8. Verbindungselement nach einem der vorangehenden Ansprüche, **dadurch gekennzeichnet, dass** die Mutter (8, 8') einen Bund (46) zum Eingriff in eine Öffnung (50) des Pedikelschraubenkopfes (52) aufweist.

## Revendications

1. Elément (2) de raccordement pour fixer une tige (56) à une vis pédiculaire (10) pour un système de stabilisation de la colonne vertébrale humaine, comportant
- un élément (4, 4') de tension avec
- une première section comprenant une queue filetée (12, 12') destinée à recevoir une tête d'une vis pédiculaire, et
- une seconde section (14, 14') présentant une ouverture (18, 18') pour recevoir et serrer ladite tige,
- un manchon (6) agencé de façon coulissante sur ladite seconde section (14),
- ledit manchon présentant une ouverture traversante (28) pour ladite queue filetée et présentant deux ouvertures (42) opposées l'une à l'autre, ou deux évidements (42'), fermées vers la queue pour recevoir ladite tige, et
- un écrou (8, 8') pouvant être vissé sur la queue pour fixer le manchon (6, 6') à la tête (52) de la vis pédiculaire (10),
**caractérisé en ce que** l'ouverture traversante (28) du manchon (6, 6') pour la queue est plus petite que les dimensions transversales de la seconde section de l'élément de tension.

2. Elément de raccordement selon la revendication 1, **caractérisé en ce que** la seconde section (14) présente une ouverture de réception circulaire.

3. Elément de raccordement selon la revendication 1, **caractérisé en ce que** la seconde section (14') présente un évidement (18') ouvert transversalement par rapport à l'axe (20') de la queue.

4. Elément de raccordement selon l'une des revendications précédentes, **caractérisé en ce que** les évidements (42') du manchon (6') sont ouverts vers l'extrémité libre du manchon.

5. Elément de raccordement selon l'une des revendications précédentes, **caractérisé en ce que** le manchon (6, 6') comporte une denture (30) autour de l'ouverture traversante (28) pour la queue (12, 12'), destinée à entrer en prise avec une denture (54) de la tête (52) de la vis pédiculaire.

6. Elément de raccordement selon l'une des revendications précédentes, **caractérisé en ce que** le manchon (6, 6') est de section transversale ronde.

7. Elément de raccordement selon l'une des revendications précédentes, **caractérisé en ce que**, dans la région (16) de la transition vers la queue (12, 12'), la seconde section (14, 14') comporte une région préférentiellement inclinée vers l'intérieur qui constitue une face d'arrêt pour une surface intérieure (38) du manchon (6, 6').

8. Elément de raccordement selon l'une des revendications précédentes, **caractérisé en ce que** l'écrou (8, 8') comporte une collerette (46) destinée à s'engager dans une ouverture (50) de la tête (52) de la vis pédiculaire.
